# EUROPEAN PATENT SPECIFICATION

(11) **EP 2 890 339 B1**
(45) Date of publication and mention of the grant of the patent: **16.01.2019**
(21) Application number: 12816148.6
(22) Date of filing: 24.10.2012
(51) Int. Cl.: A61F 5/11, A61F 13/10, A61F 13/02, A61F 13/06

(54) **KIT FOR CORRECTING AND PREVENTING INGROWN NAILS AND INSTRUCTION FOR ITS USE**
KIT ZUR KORREKTUR UND VERHINDERUNG EINGEWACHSENER NÄGEL UND ANLEITUNG ZU SEINER VERWENDUNG
TROUSSE POUR CORRIGER ET PRÉVENIR DES ONGLES INCARNÉS ET INSTRUCTION POUR SON UTILISATION

(30) Priority: 02.11.2011 SI 201100415
(43) Date of publication of application: 08.07.2015
(73) Proprietor: Snoj, Zvezdana, 1000 Ljubljana (SI)
(72) Inventor: Snoj, Zvezdana, 1000 Ljubljana (SI)
(74) Representative: Müller Hoffmann & Partner
(86) International application number: PCT/SI2012/000058
(87) International publication number: WO 2013/066275

(56) References cited:
- WO-A1-2008/105166
- WO-A1-2011/002929
- US-A- 3 464 408
- US-A- 3 799 160
- US-A1- 2007 287 945

## Description

The subject of the invention is a kit for correcting and preventing ingrown nails, particularly on the feet, where the nail most frequently affected is on the big toe. The kit that is the subject of the invention comprises a disinfectant and a specially designed plaster.

The kit that is the subject of the invention is intended for the conservative, non-surgical treatment of ingrown nails, and for the prevention thereof. The invention belongs to the Class A 61f05/00 of the International Patent Classification.

Ingrown nails most frequently occur on the big toe, but can also occur on other nails. The nail in this extremely painful condition cuts into the paronychium or nail bed, damaging it and causing injury. The wound frequently becomes infected, with the skin becoming highly inflamed. Purulent granulation tissue appears in the fold between the skin and nail, producing pus and extreme pain that limits or makes walking and movement impossible.

An ingrown nail occurs when part of the nail cuts into the skin and nail bed tissue. An ingrown nail in the nail bed tissue is a frequent problem that primarily occurs as a long-term, recurring chronic condition. The causes of ingrown nails are numerous, and are most frequently encountered in encountered in young children and adolescents, diabetics, persons suffering from hyperhidrosis (excessive sweating) of the feet, athletes and men. Some persons also demonstrate a disposition to this condition. The occurrence of ingrown nails can sometimes be linked to the improper cutting of nails, while other factors also exist, such as overly tight footwear, which causes an injury that frequently becomes infected. Typically, one side of the nail cuts deeply into the nail bed, such that the edge of the nail is not visible. The edge of the nail thus cuts into the skin and tissue, damaging it.

The currently accepted method for correcting an ingrown nail is surgery that requires the removal of the internal or external quarter of the nail with the associated nail bed tissue and nail matrix, or the entire nail itself. Following excision, the edges of the wound are stitched. If the infection is extremely purulent, the wound must be left wide open. A variation of the surgical procedure for correcting an ingrown nail is described in patent document no. RU 2403877.

In addition to the aforementioned surgical procedure, various devices and instruments have been developed to correct and prevent ingrown nails, particularly on the feet. Patent document JP 2011104231 describes one of those solutions, in which a shaped metal plate is placed under the ingrown nail. A wire attached to one end of the plate on the other side of the nail is pulled gradually to straighten the ingrown nail. This type of solution is above all painful, and does permit the wearing of footwear during the treatment of the ingrown nail. Corrective pre-stressed bands (as described in patent document KR 20110018149) work in a similar manner. The bands are attached to the nail in their pre-stressed state and pull the nail outwards when released. The majority of these solutions are either complicated to use, and consequently expensive, or have little practical value.

Technological advances for resolving the problem of ingrown nails also address the theory that the problem can be resolved simply by reshaping the nail. Thus, patent document US 3,981,298 describes a system in which a liquid substance used for ingrown nails hardens and subsequently serves to maintain their shape. The aforementioned invention includes the use of a band that is placed over the entire surface of the nail. However, this system merely reshapes the nail, and does not address the cycle of inflammation and the injuries to the nail that occur in the area of inflammation. Patent document US 5,862,811 describes a system that includes glues and solvents that fill the cracks in a nail with polystyrene pellets. Neither solution solves the problem of the injured nail bed. They merely attempt to shape the nail so that it does not grow inwards. Patent document US 6,095,995 emphasises the use of a shape-memory alloy or resinous compound that is shaped to match the curvature of nails at room temperature and becomes straight when heated. Similar to the previously mentioned inventions, the above solution only addresses the reshaping of nails. Thus, the need still remains for a system that isolates the sharp edge of the nail and provides a smooth surface for the outside edge of the nail to reduce the possibility of inflammation, infection, injuries and pain.

US 3 464 408 A discloses a triangular bandage for relieving ingrown toenails. The bandage has converging sides which are adapted to slide under the corners of ingrown toenails. The bandage is made of a gauze-like material.

US 3 799 160 A discloses a device and a method for correcting ingrown toenails with a flexible self-supporting nail support in the form of a thin flat plate.

From WO 2011/002929 A1, an antimicrobial and preservative composition is known.

US 2007/0287945 A1 discloses an ingrown toenail treatment method having a cotton ball which is guided with a tooth pick to an affected area of the toe.

It is an object of the present invention to improve the structure and use of a bandage for relieving ingrown toenails.

The object is solved by a kit for correcting and preventing ingrown nails according to claim 1 and 2.

The invention's ingrown toenail correction and prevention kit contains a disinfectant and a porous adhesive tape with a two-sided, non-adhesive foil that is inserted directly between the ingrown nail and the affected tissue.

We will explain the kit in more detail based on the example presented and the following figures:

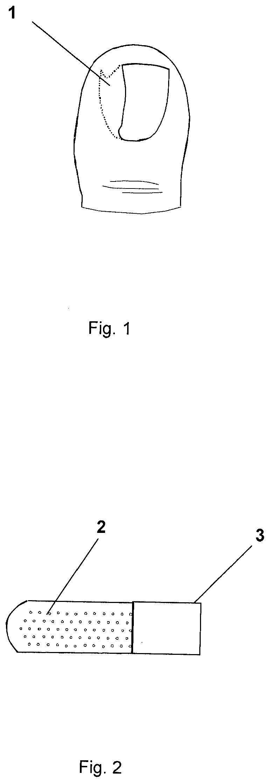

- Figure 1:: illustration of an affected nail,
- Figure 2:: illustration of a plaster,
- Figure 3:: illustration of a disinfectant,
- Figure 4:: illustration of the placement of the plaster on the affected area, and

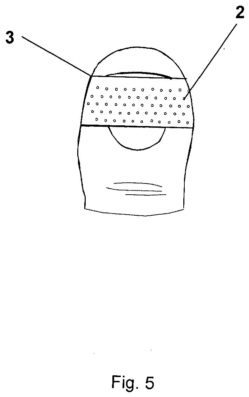

- Figure 5:: illustration of the nail with the plaster in place.

According to the herein disclosed procedure instructions, the plaster in the ingrown toenail correction and prevention kit is to be directly inserted between the ingrown nail and the affected tissue, such that it physically separates the hail from the affected tissue.

The plaster in the kit that is the subject of the invention comprises a porous adhesive tape (2) with a two-sided, non-adhesive foil (3), of a specific thickness and hardness, on one end. The herein disclosed plaster fixation procedure also includes a disinfectant (4) in the form of a spray is used during the placement of the plaster. The disinfectant (4) is based on 2-propanol, chlorhexidine-gluconate and hydrogen peroxide.

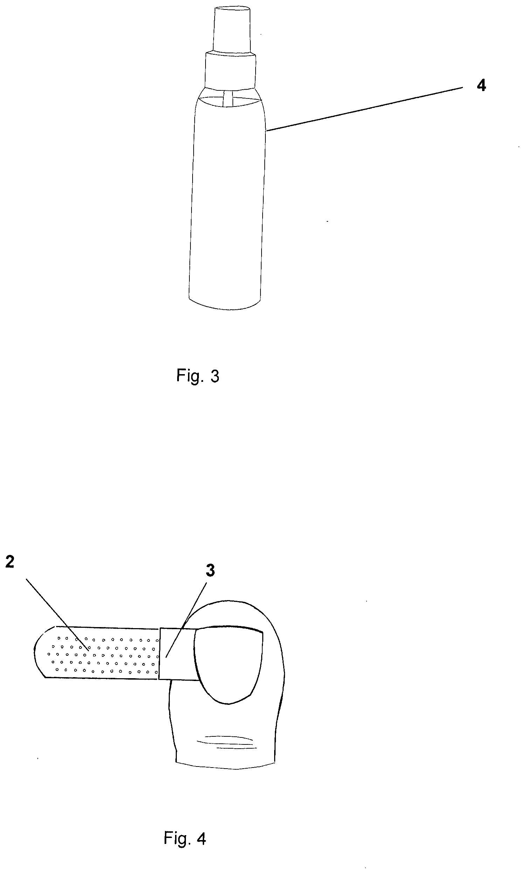

The procedure for applying a plaster to correct and prevent ingrown nails is shown in Figure 4. We first disinfect the affected area of the ingrown nail (1) using the disinfectant (4). We place the invention's plaster with the two-sided, non-adhesive foil (3) such that the two-sided, non-adhesive foil (3) is placed between the ingrown nail and the affected tissue. We then wrap the toe (or finger), on which the portion of the plaster with the two-sided, non-adhesive foil has been placed between the ingrown nail and the affected tissue, with porous adhesive tape (2) which, due to it porous nature, does not close the wounded tissue hermetically and allows the wound to "breath".

The kit that is the subject of the invention is, which comprises a plaster to correct and prevent ingrown nails that comprises a porous adhesive tape (2) with a two-sided, non-adhesive foil (3), and a disinfectant (4), is simple, easy and safe to use for both adults and children. A kit designed as such is appropriate for use at home as a self-help aid for ingrown nails, and for use in outpatient clinics and hospitals. The use of such a kit is also less expensive than other invasive methods, as the patient is able to walk while making use of the kit, while no sick-leave time is required. This means a less expensive way to correct and prevent ingrown nails.

## Claims

1. Kit for correcting and preventing ingrown nails,
wherein the kit contains
- a plaster comprising a porous adhesive tape (2) with a two-sided, non-adhesive foil (3) on one end, and
- a disinfectant (4);
**characterized in that**
- the tape (2) has a longitudinal extension and the non-adhesive foil (3) is arranged on the one end of the tape (2) in the longitudinal direction of the tape (2).

2. The kit according to claim 1,
**characterized in that**
the disinfectant (4) is based on 2-propanol, chlorhexidine-gluconate and hydrogen peroxide.

## Patentansprüche

1. Set zum Korrigieren und Verhindern eingewachsener Nägel, wobei das Set enthält:
ein Pflaster mit einem porösen Klebeband (2) mit einer beidseitigen, nicht klebenden Folie (3) auf einem Ende, und
ein Desinfektionsmittel (4);
**dadurch gekennzeichnet, dass**
das Band (2) ein longitudinale Ausdehnung hat und die nicht klebende Folie (3) auf dem einen Ende des Bands (2) in der longitudinalen Richtung des Bands (2) angeordnet ist.

2. Set nach Anspruch 1,
**dadurch gekennzeichnet, dass**
das Desinfektionsmittel (4) auf 2-Propanol, Chlorhexidingluconat und Wasserstoffperoxid basiert.

## Revendications

1. Trousse pour corriger et prévenir des ongles incarnés, la trousse contenant :
- un pansement comprenant un ruban adhésif poreux (2) avec un film non adhésif double face (3), sur une extrémité, et
- un désinfectant (4) ;
**caractérisée en ce que**
- le ruban (2) a une extension longitudinale, et le film non adhésif (3) est disposé sur ladite extrémité du ruban (2), dans le sens longitudinal dudit ruban (2).

2. Trousse selon la revendication 1, **caractérisée en ce que** le désinfectant (4) est à base de 2-propanol, de gluconate de chlorhexidine et de peroxyde d'hydrogène.
